# EUROPEAN PATENT APPLICATION

(11) **EP 2 131 237 A1**
(43) Date of publication of application: **09.12.2009**
(21) Application number: 09161488.3
(22) Date of filing: 29.05.2009
(51) Int. Cl.: G03B 9/04, A61B 1/04

(54) **Light controlling apparatus**

(30) Priority: 04.06.2008 JP 2008146599
(71) Applicant: Olympus Corporation, Shibuya-ku Tokyo 151-0072 (JP)
(72) Inventor: Okita, Tatsuhiko, Tokyo 151-0072 (JP)
(74) Representative: Winter, Brandl, Fürniss, Hübner Röss, Kaiser, Polte Partnerschaft Patent- und Rechtsanwaltskanzlei

(57) **Abstract**

For providing a small-size light controlling apparatus in which a desirable opening and closing operation of a diaphragm blade (301) is possible by driving an ion-conducting actuator (501) by a small voltage, a light controlling apparatus is made to include a substrate (101) in which an optical aperture is formed (102), a light controlling mechanism (301) which is provided with another optical aperture (302) and a shielding section, an actuator (501) which generates a power which rotates the light controlling mechanism, a controlling mechanism which displaces the actuator, and a transmission member (401) of which, one end is coupled with the light controlling mechanism, and the other end is coupled with the actuator via a coupling member (503), and which transmits the power of the actuator to the light controlling mechanism. The transmission member, at the time of transmitting the power, widens the displacement of the actuator.

## Description

### BACKGROUND OF THE INVENTION

### Field of the Invention

The present invention relates to a small-size light controlling apparatus, and particularly to a light controlling apparatus which is suitable for an endoscope of a thin diameter.

### Description of the Related Art

In an endoscope apparatus in which a solid image pickup element is used, in recent years, with a progress of a semiconductor manufacturing technology, it has been possible to use a solid image pickup element having fine pixels.

However, the solid image pickup element having the fine pixels is sensitive to a change of a focal position due to a distance up to an object to be photographed. In an endoscope in which an optical system of a conventional fixed focus and a fixed diaphragm is used, even when the image pickup element having the fine pixels is used, it has been difficult to achieve a high-definition image.

To solve this problem, the following methods may be taken into consideration. A method of adding a focus adjustment function by making a lens to be displaced is available. Moreover, as another method, a method of achieving a favorable imaging by making small an aperture diameter at the time of near-point photography and increasing a depth of focus, by adjusting such that an optical system is capable of having an appropriate imaging for a far-point photography object at a fixed focus is available.

In a case of the latter method, a variable aperture mechanism is necessary, and there is a problem that at the time of near-point photography, an amount of light reaching the solid image pickup element decreases. In an endoscope, since photographs are taken by light of a lighting system at a front-end portion normally, it is possible to secure sufficient amount of light at the time of near-point image pickup, and it is not a major problem.

In this manner, to make the most of such capability of the image pickup element of the fine pixels, a mechanism such as a lens driving unit and a variable aperture unit is necessary. However, it is difficult to incorporate the lens driving unit in the endoscope of a thin diameter. Therefore, it is desirable to use an ultra-small variable aperture unit in the endoscope of a thin diameter. As an example of the ultra-small variable aperture unit light controlling apparatus which can be used in the endoscope, an ultra-small variable aperture unit in which an ion-conducting actuator is used has been provided in Japanese Patent Application Laid-open Publication No. 2007-127699.

In an invention disclosed in Japanese Patent Application Laid-open Publication No. 2007-127699, a structure is such that the ion-conducting actuator is coupled directly with a diaphragm blade via a drive shaft. Therefore, a substantial displacement of the ion-conducting actuator has been necessitated for opening and closing the diaphragm blade. Here, there is a possibility of decline in an amount of displacement of the ion-conductive actuator by supplying an electric power repeatedly. This tendency is particularly remarkable when an attempt is made to achieve the substantial amount of displacement by applying a high voltage.

For solving this problem, it is necessary to add to the aperture unit a function which enables to widen the displacement of the actuator. Incidentally, when such a function is added, there is an increase in a size of the aperture apparatus.

### SUMMARY OF THE INVENTION

The present invention is made in view of the abovementioned circumstances, and an object of the present invention is to provide an ultra-small light controlling apparatus in which an opening and closing operation of a diaphragm blade is possible by a small displacement of an actuator.

To solve the abovementioned issues, and to achieve the object, according to the present invention, there can be provided a light controlling apparatus including
a substrate in which, an optical aperture is formed,
a light controlling mechanism which is provided with another optical aperture and a shielding section,
an actuator which generates a power which rotates the light controlling mechanism,
a controlling mechanism which displaces the actuator, and
a transmission member of which, one end is coupled with the light controlling mechanism, and the other end is coupled with the actuator via a coupling member, and which transmits the power of the actuator to the light controlling mechanism, and
the transmission member, at the time of transmitting the power, widens the displacement of the actuator.

According to a preferable aspect of the present invention, it is desirable that the transmission member is disposed between an outer circumferential portion of the optical aperture formed in the substrate and an outer circumferential portion of the substrate.

Moreover, according to a preferable aspect of the present invention, it is desirable that both ends of the transmission member are disposed at positions face-to-face, sandwiching the optical aperture formed in the substrate.

Furthermore, according to a preferable aspect of the present invention, it is desirable that all components other than the actuator, the controlling mechanism, and the coupling member are a stacked structure made of a plating layer.

According to a preferable aspect of the present invention, it is desirable that a frame member has an aperture which is larger than the optical aperture formed in the substrate, and stacked structure is covered by the frame member which is larger than an outer diameter of the substrate.

Moreover, according to a preferable aspect of the present invention, it is desirable that the actuator is an actuator which has a circular arc shape formed by an elastic member, and which changes a chord length thereof by the controlling mechanism, and that the transmission member with which the actuator is coupled is driven by the change in the chord length of the actuator, and the light controlling apparatus which is coupled with the transmission member is rotated.

Furthermore, according to a preferable aspect of the present invention, it is desirable that by the rotation of the light controlling mechanism having the other optical aperture, the other optical aperture moves to a first stationary position which overlaps with a position of the optical aperture formed in the substrate, and a second stationary position which is a position retracted from the position of the optical aperture formed in the substrate, and switches to the optical aperture formed in the substrate and the other optical aperture formed in the light controlling mechanism.

According to a preferable aspect of the present invention, it is desirable that the actuator having the circular arc shape is formed of a high-molecular material containing ions, and includes a pair of electrodes, on a surface of a central side of the circular arc, and on a surface facing the surface of the central side of the circular arc, and that a voltage is applied between the pair of electrodes by the controlling mechanism, and the chord length thereof is changed by moving the ions.

### BRIEF DESCRIPTION OF THE DRAWINGS

Fig. 1 is a diagram showing an exploded view of a light controlling apparatus according to a first embodiment of the present invention;
Fig. 2 is a diagram showing an assembly diagram of the light controlling apparatus according to the first embodiment;
Fig. 3 is a diagram explaining an operation of an ion-conducting actuator;
Fig. 4 is a diagram showing a process of manufacturing each layer of a stacked structure;
Fig. 5 is a diagram in which various layers of the stacked structure are stacked;
Fig. 6 is a diagram showing a state in which a sacrificing layer is removed by an etching, in a state in Fig. 5;
Fig. 7 is a diagram explaining an operation of a fist stationary position of the first embodiment;
Fig. 8 is a diagram explaining an operation of a second stationary position in the first embodiment;
Fig. 9 is a diagram explaining an overall operation when a voltage is applied, in the first embodiment;
Fig. 10 is a diagram explaining an overall operation when applying of voltage is stopped, in the first embodiment;
Fig. 11 is a diagram showing an exploded view of a light controlling apparatus according to a second embodiment of the present invention;
Fig. 12 is a diagram showing an assembly diagram of the light controlling apparatus according to the second embodiment;
Fig. 13 is a diagram explaining an operation of a first stationary position of the second embodiment;
Fig. 14 is a diagram explaining an operation of a second stationary position of the second embodiment;
Fig. 15 is diagram explaining an overall operation when a voltage is applied, in the second embodiment; and
Fig. 16 is a diagram explaining an overall operation when applying of voltage is stopped, in the second embodiment.

### DETAILED DESCRIPTION OF THE INVENTION

Exemplary embodiments of a light controlling apparatus according to the present invention will be described below in detail by referring to the accompanying diagram. However, the present invention is not restricted by the embodiments described below.

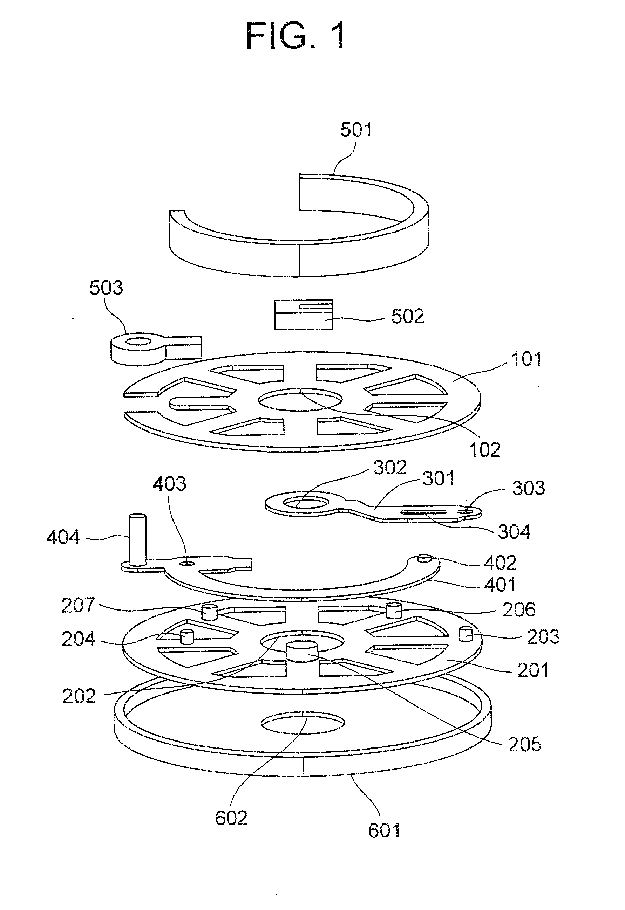

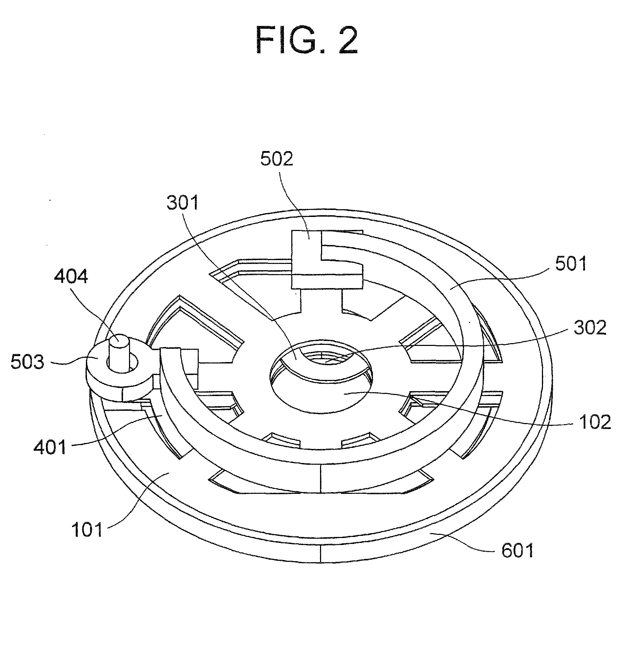

A light controlling apparatus of a first embodiment will be described below by using diagrams from Fig. 1 to Fig. 10. Fig. 1 shows an exploded view of the light controlling apparatus, and Fig. 2 shows an assembly diagram of the light controlling apparatus.

As shown in Fig. 1, the light controlling apparatus includes a first substrate 101 in which a first aperture 102 is formed, a second substrate 201 in which a second aperture 202 which is larger than the first aperture 102, rotating shafts 203 and 204, and spacers 205, 206, and 207 are formed, a light controlling means (a light controlling mechanism) 301 in which a third aperture 302 which is smaller than the first aperture 102 and the second aperture 202, a drive shaft hole 304, and a rotating shaft hole 303 are formed, a transmission member 401 in which a rotating shaft hole 403 and a drive shafts 402 and 404 are formed, and an ion-conducting actuator 501 of which, one end is fixed to an electrode 502 which is fixed by adhering on the first substrate 101, and the other end is fixed by adhering to a coupling member 503.

The rotating shaft 203 formed on the second substrate 201 is joined to the first substrate 101 via the rotating shaft hole 303 formed in the light controlling means 301. The rotating shaft 204 is joined to the first substrate 101 via the rotating shaft hole 403 formed in the transmission member 401. The drive shaft 402 formed in the transmission member 401 is inserted into the drive shaft hole 304 formed in the light controlling means 301, and the spacers 205 to 207 formed on the second substrate 201 are joined to the first substrate 101 directly.

Moreover, the drive shaft 404 formed on the transmission member 401 as shown in Fig. 2 is coupled with the ion-conducting actuator 501 by the coupling member 503. Furthermore, the first substrate 101 and the second substrate 201 which are coupled, the light controlling means 301, and the transmission member 401 are covered entirely by a frame member 601 having an aperture 602 which is larger than the first aperture 102.

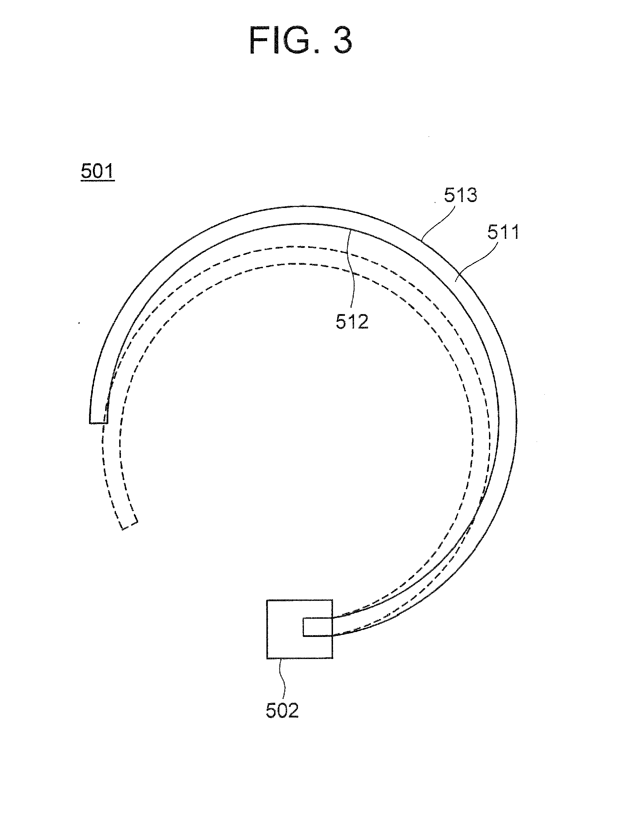

Next, a detail diagram of the ion-conducting actuator 501 is shown in Fig. 3. The circular arc shaped ion-conducting actuator 501 has a three-layered structure of an ion-containing polymer 511 which is a circular arc substrate, a first electrode 512 which is provided on a surface of a central side of the circular arc, and a second electrode 513 which is provided on a surface facing the surface of the central side of the circular arc.

Here, an electric potential difference is imparted between the first electrode 512 and the second electrode 513 by outputting a voltage from an external voltage source. Cations of the ion-containing polymer 511 move to a cathode side. As a result, the cathode side of the ion-conducting polymer 511 swells, and a curvature of the circular arc shape is changed as shown by dotted lines in Fig. 3, which results in a change in a chord length. In this manner, it is possible to change the chord length of the ion-conducting actuator 501 in a predetermined range by an output voltage from the external voltage source.

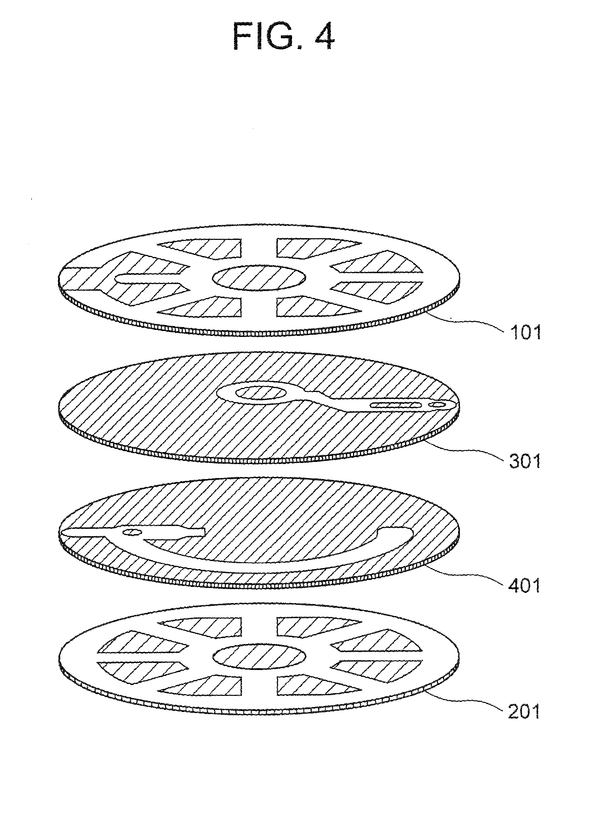

Next, in the light controlling apparatus, a manufacturing process of a stacked structure excluding the ion-conducting actuator 501, the electrode 502, the coupling member 503, and the frame member 601 is shown in Fig. 4, Fig. 5, and Fig. 6. In the description of the manufacturing process, for making the manufacturing process easily understandable, diagrams in which the drive shafts 402 and 404, the rotating shafts 203 and 204, and the spacers 205, 206, and 207 are omitted are used.

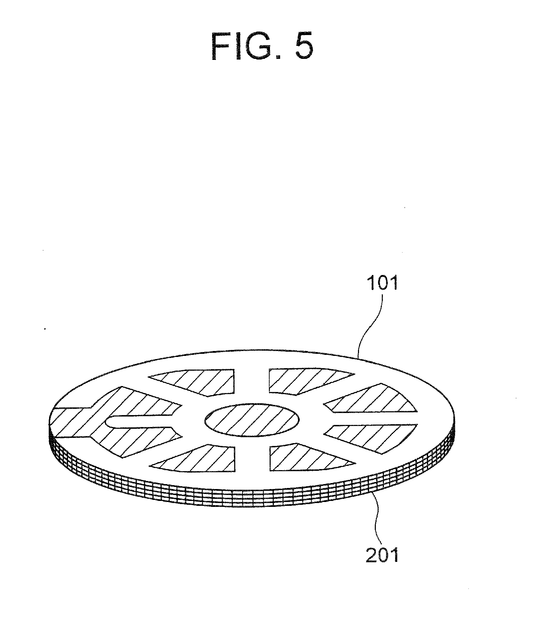

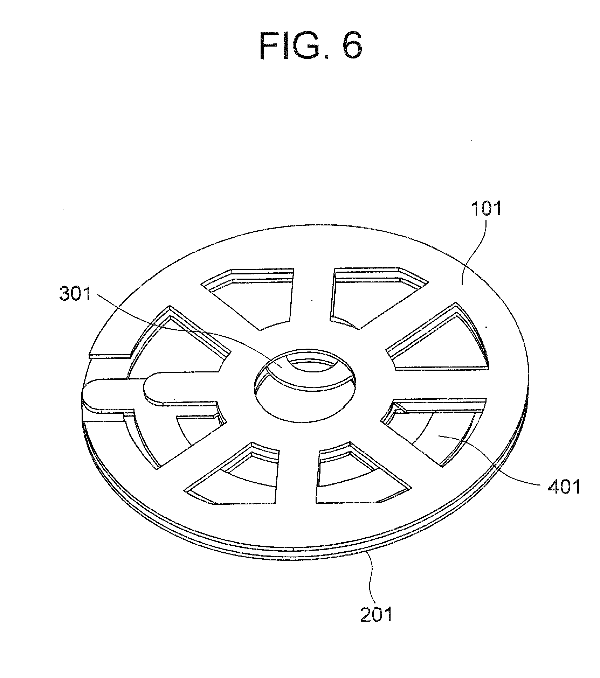

The stacked structure is manufactured by using a plating process and an etching process. As shown in Fig. 4, each layer is made of a plating layer or plated layer which is a stacked structure, and a sacrificing layer which is removed by the etching after the stacking. In Fig. 4, each layer is shown to be disassembled such that each layer is clearly visible. Essentially, various layers are stacked in order of a layer forming the second substrate 201, a sacrificing layer which is to be removed later, a layer of the transmission member 401, a sacrificing layer which is to be removed layer, a layer forming the light controlling means 301, a sacrificing layer which is to be removed later, and a layer forming the first substrate 101. Fig. 5 shows a diagram in which various layers are stacked. The stacked structure is formed as shown in Fig. 6 by removing the sacrificing layer formed in each layer, by the etching, after various layers are stacked.

A merit of using this process is described below. First of all, it is possible to manufacture a fine structure with a high machining accuracy. Next, it is possible to manufacture even a complicated structure by letting the structure to be a multi-layered structure. Furthermore, this process could adopt a self-assembly, therefore an assembling is not necessary. Moreover, a batch process is possible. By using this process, as in this means, even when the structure is complicated, the means doesn't become large.

However, for removing the sacrificing layer by the etching, a space is to be formed in each layer. When the space is formed, light is incident also from openings other than the first aperture 102 and the second aperture 202 formed in the first substrate 101 and the second substrate 201 respectively. Therefore, for using this stacked structure as the light controlling apparatus, by fitting the stacked structure in the frame member 601 shown in Fig. 1, and shielding the apertures other than the first aperture 102 and the second aperture 202 formed in the first substrate 101 and the second substrate 201, it is possible to prevent the light from passing through the openings other than the first aperture 102 and the second aperture 202.

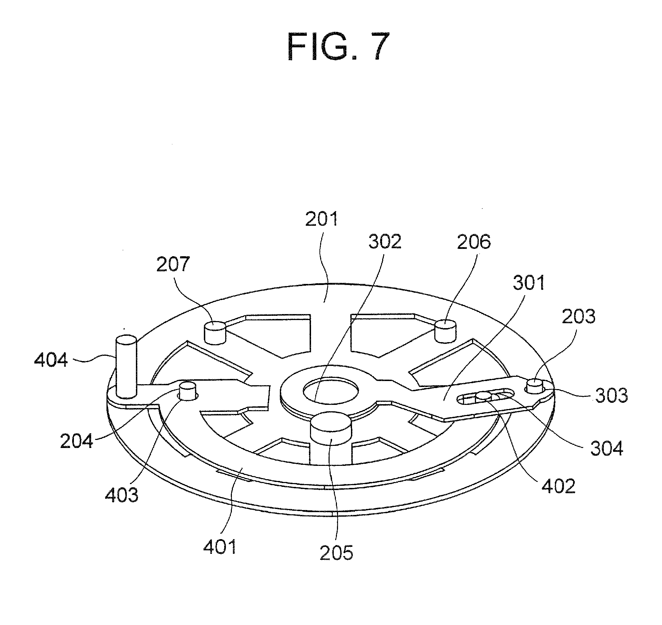

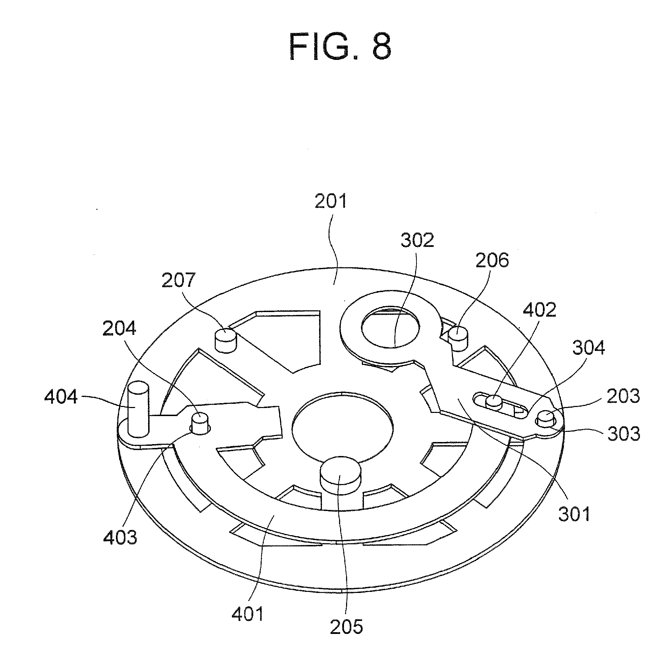

An operation of each component will be described below in detail by using Fig. 7 and Fig. 8. Fig. 7 and Fig. 8 are diagrams in which, the first substrate 101, the ion-conducting actuator 501, the electrode 502, the coupling member 503, and the frame member 601 are omitted to make the diagrams easily understandable.

As shown in Fig. 7 and Fig. 8, the transmission member 401 is rotatable around the rotating shaft 204 as a center, which is formed in the second substrate 201. Moreover, the light controlling means 301 is rotatable around the rotating shaft 203 as a center. The transmission member 401 and the light controlling means 301 are coupled by the drive shaft 402 formed on the transmission member 401. By moving the drive shaft 404 formed on the transmission member 401, the transmission member 401 rotates, and with the rotation of the transmission member 401, the light controlling means 301 also rotates.

As shown in Fig. 7, when the light controlling means 301 makes a contact with the spacer 205 upon coming to a first stationary position overlapping with the second aperture 202, the light controlling means 301 stops at that position. At this time, the third aperture 302 formed in the light controlling means 301 becomes an optical aperture of the light controlling apparatus. Moreover, as shown in Fig. 8, when the light controlling means 301 rotates and comes to a second position which is a position retracted completely from the second aperture 202, the light controlling means 301 makes a contact with the spacer 206, and stops at that position.

It is not shown in the diagram, but at this time, the first aperture 102 formed in the first substrate 101 becomes the optical aperture of the light controlling apparatus. In this manner, the optical aperture is switched by the rotation of the light controlling means 301.

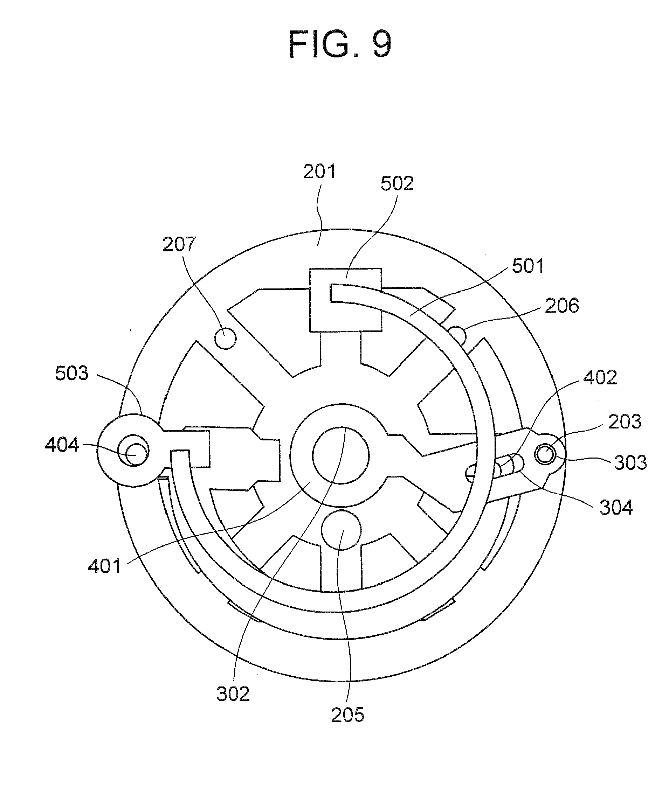

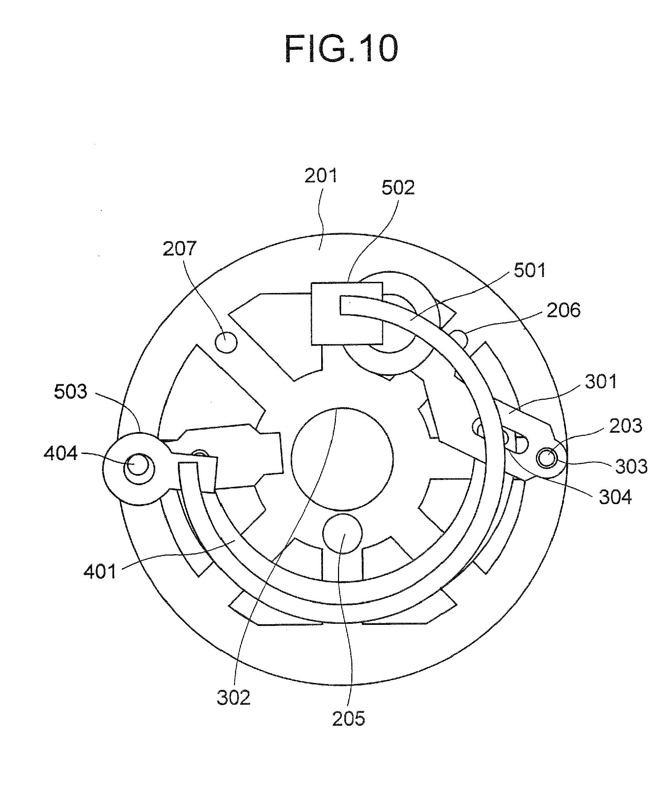

Next, an operation of the light controlling apparatus according to the first embodiment will be described below by using Fig. 9 and Fig. 10. Fig. 9 and Fig. 10 are top views, and the first substrate 101 is omitted in Fig. 9 and Fig. 10 to make the operation of the mechanism easily understandable.

Fig. 9 is a diagram showing a state in which, the light controlling means 301 makes a contact with the spacer 205, and is at the first stationary position overlapping with the second aperture 202. The third aperture 302 formed in the light controlling means 301 becomes the optical aperture of the light controlling apparatus in this state. Moreover, the ion-conducting actuator 501 is fixed to the electrode 502, and is coupled with the drive shaft 404 formed on the transmission member 401, via the coupling member 503.

Moreover, as shown in Fig. 10, by supplying an electric power to the ion-conducting actuator 501 by the electrode 502, the curvature of the circular arc shape changes, and as a result, (the ion-conducting actuator 501) is displaced. The transmission member 401 coupled with the ion-conducting actuator 501 via the coupling member 503 rotates around the rotating shaft 204 formed on the second substrate 201, as a center, and the light controlling means 301 coupled with the drive shaft 402 formed on the transmission member 401 rotates around the rotating shaft 203 formed on the second substrate 201, as a center. Rotational movement of the light controlling means 301 which has rotated, stops when the light controlling means 301 makes a contact with the spacer 206, and the light controlling means 301 stops at the second position which is a position retracted completely from the second aperture 202. In this state, although it is not shown in the diagram, the first aperture 102 formed in the first substrate 101 becomes the optical aperture of the light controlling apparatus.

In the first embodiment, a front end of the coupling member 503 is formed to be ring-shaped, and is coupled with the drive shaft 404 formed on the transmission member 401. However, it is possible to achieve a similar effect provided that the shape of the front end of the coupling member 503 is a shape which can be hooked to the drive shaft 404, such as a shape in which a notch is formed in a rectangle, and not only the ring shape.

In the first embodiment, the transmission member 401 which widens an amount of displacement of the ion-conducting actuator 501 is provided, and by coupling the transmission member 401 to the light controlling means 301, the displacement is transmitted upon increasing. Consequently, it is possible to reduce substantially the amount of displacement of the ion-conducting actuator 501 which is necessary for opening and closing the light controlling means 301. Therefore, it is possible to reduce substantially a voltage to be supplied to the ion-conducting actuator 501, and also to improve reliability of the ion-conducting actuator 501.

Moreover, in a case of manufacturing a complicated structure as in the first embodiment, with the conventional machining technology, e.g. cutting, press machining, the structure becomes large which is a problem. In the first embodiment, since the stacked structure is manufactured by using the plating process and the etching process, such fine and complicated structure is possible. Furthermore, a self assembly in which an assembling process is not necessary is possible.

### [Second embodiment]

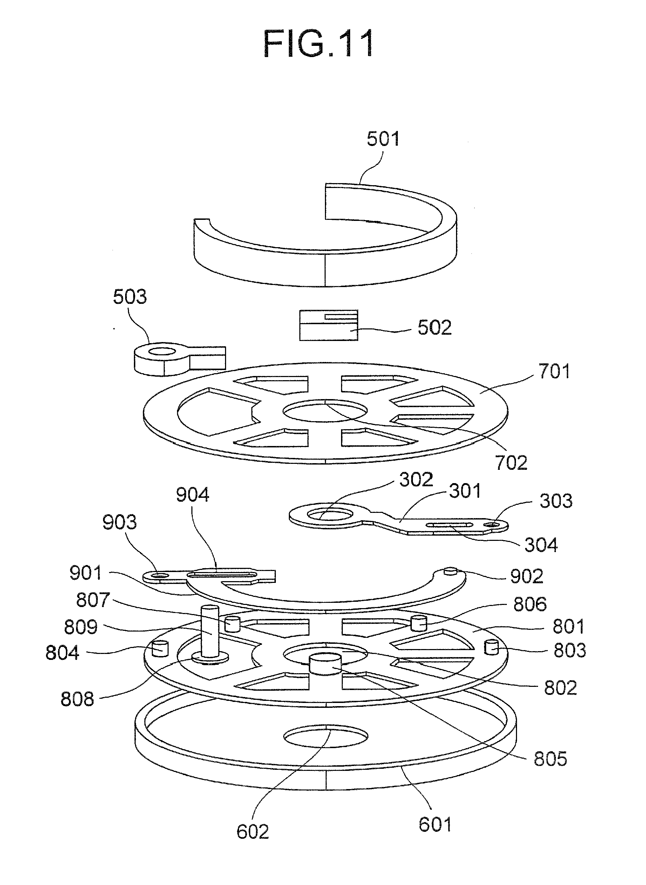

Fig. 11 is a diagram showing an exploded view of a second embodiment of the light controlling apparatus according to the present invention. The second embodiment will be described below by using diagrams from Fig. 11 to Fig. 16. Same reference numerals are assigned to components which are same as in the first embodiment, and the description of such components is omitted. A structure of the light controlling apparatus of the second embodiment will be described below.

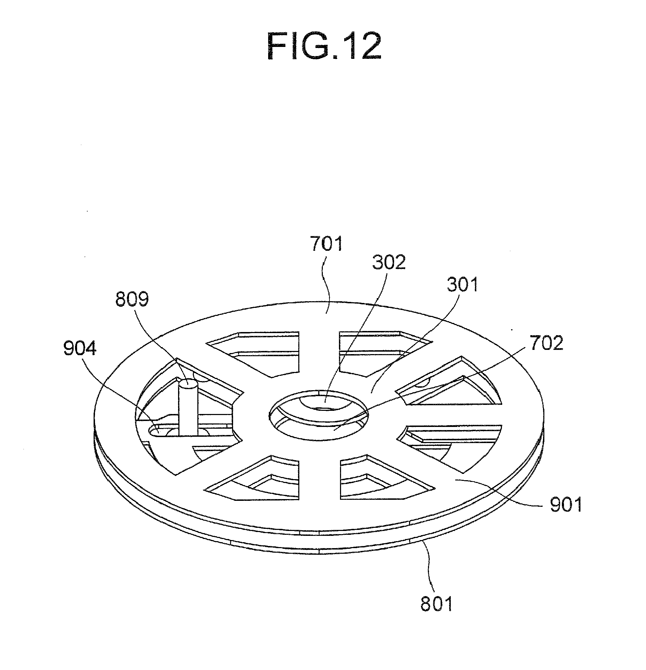

Fig. 11 is an exploded view of the light controlling apparatus. Fig. 12 is an assembly diagram in which the ion-conducting actuator 501, the coupling member 503, the electrode 502, and the frame member 601 are omitted.

As shown in Fig. 11, the light controlling apparatus includes a first substrate 701 in which a first aperture 702 is formed, a second substrate 801 in which a second aperture 802 which is larger than the first aperture 702, rotating shafts 803 and 804, and spacers 805, 806, and 807 are formed, a light controlling means 301 in which a third aperture 302 which is smaller than the first aperture 702 and the second aperture 802, a drive shaft hole 304, and a rotating shaft hole 303 are formed, a transmission member 901 in which a rotating shaft hole 903, a drive shaft 902, and a groove 904 are formed, and the ion-conducting actuator 501 of which, one end is fixed to the electrode 502 which is fixed by adhering on the substrate 701, and the other end is fixed by adhering to the coupling member 503.

The rotating shaft 803 formed on the second substrate 801 is joined to the first substrate 701 via the rotating shaft hole 303 formed in the light controlling means 301. The rotating shaft 804 is joined to the first substrate 701 via the rotating shaft hole 903 formed in the transmission member 901. Moreover, a drive shaft 809 connected to the drive bearing 808 is provided independently in a layer of the second substrate 801. The drive shaft 809 is protruded on the first substrate 701 via the groove 904 formed in the transmission member 901.

The spacers 805, 806, and 807 formed on the second substrate 801 are joined directly to the first substrate 701. Moreover, it is not shown in the diagram but the drive shaft 809 protruded from the first substrate 701 is coupled with the ion-conducting actuator 501 by the coupling member 503. Furthermore, similarly as in the first embodiment, the first substrate 701, the second substrate 801, the light controlling means 301, and the transmission member 901 which are coupled are covered entirely by the frame member 601 having the aperture 602 lager than the first aperture 702.

The description is omitted in the second embodiment, but components other than the ion-conducting actuator 501, the electrode 502, the coupling member 503, and the frame member 601 form a stacked structure made of a plated layer, which is manufactured by using the plating process and the etching process, similarly as in the first embodiment.

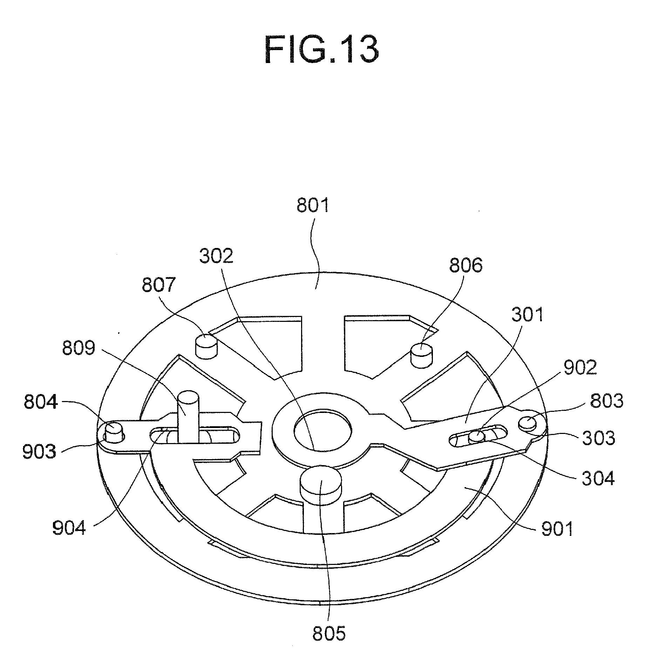

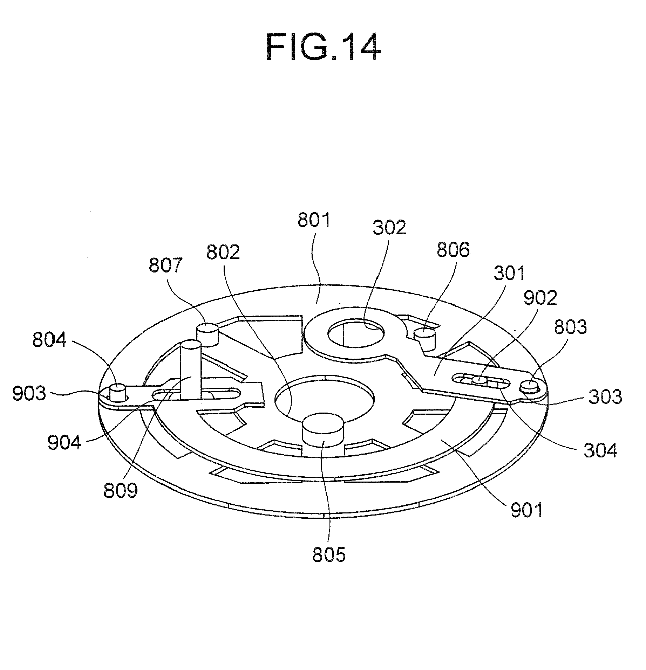

Each component will be described below in detail by using Fig. 13 and Fig. 14. Fig. 13 and Fig. 14 are state diagrams in which the first substrate 701, the ion-conducting actuator 501, the electrode 502, the coupling member 503, and the frame member 601 are omitted for making the diagrams easily understandable.

As shown in Fig. 13 and Fig. 14, the transmission member 901 is rotatable around the rotating shaft 804 as a center, which is formed in the second substrate 801. The light controlling means 301 is rotatable around the rotating shaft 803 as a center. The transmission member 901 and the light controlling means 301 are coupled by the drive shaft 902 formed in the transmission member 901. Moreover, the drive shaft 809 which is protruded from the groove 904 formed in the transmission member 901 is movable along the groove 94. In this manner, by operating the drive shaft 809, the transmission member 901 rotates, and with the rotation of the transmission member 901, the light controlling means 301 also rotates and switches the optical aperture.

As shown in Fig. 13, when the light controlling means 301 makes a contact with the spacer 805 upon coming to a first stationary position overlapping with the second aperture 802, the light controlling means 301 stops at that position. At this time, the third aperture 302 formed in the light controlling means 301 becomes an optical aperture of the light controlling apparatus. Moreover, as shown in Fig. 14, when the light controlling means 301 rotates and comes to a second position which is a position retracted completely from the second aperture 802, the light controlling means 301 makes a contact with the spacer 806, and stops at that position. It is not shown in the diagram, but at this time, the first aperture 702 formed in the first substrate 701 becomes the optical aperture of the light controlling apparatus. In this manner, the transmission member 901 rotates, and with the rotation of the transmission member 901, the light controlling means 301 also rotates, thereby switching the aperture.

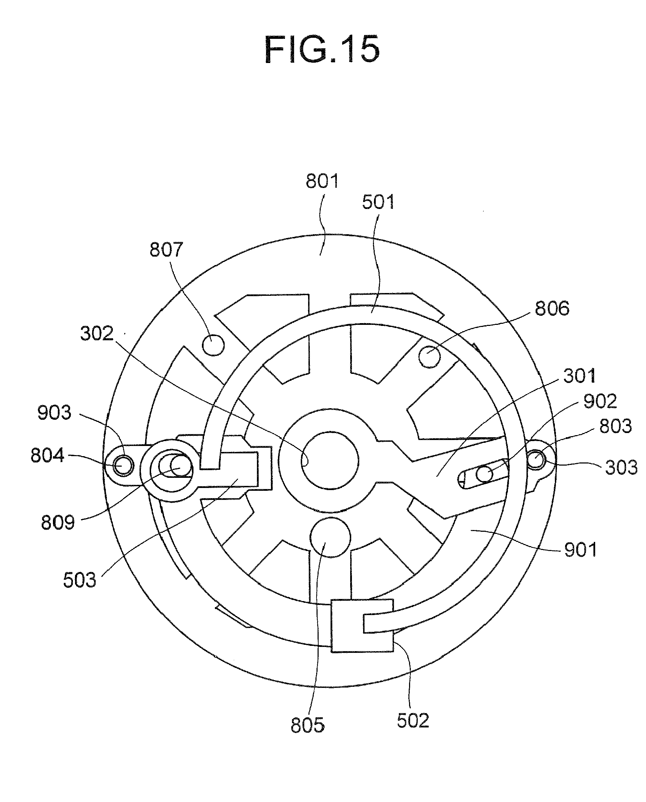

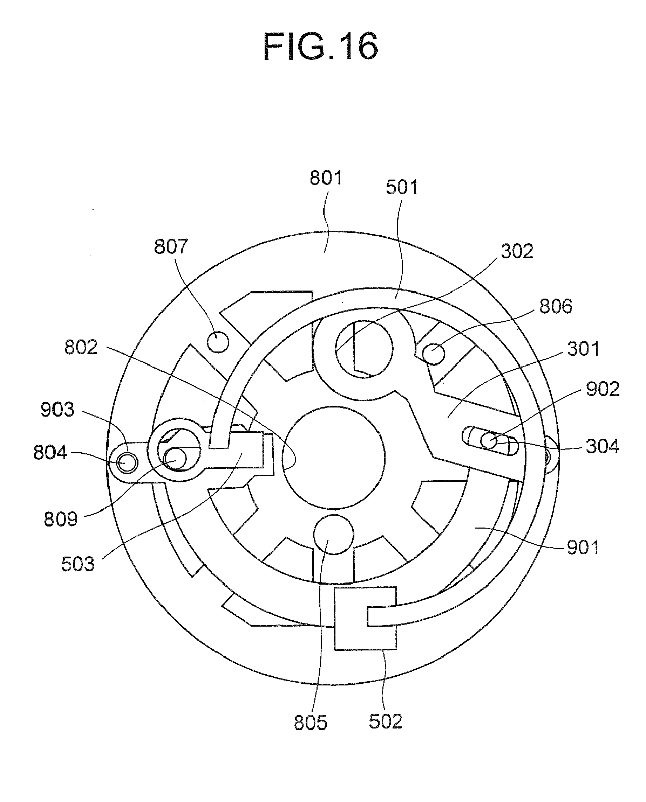

Next, an operation of the light controlling apparatus according to the second embodiment will be described below by using Fig. 15 and Fig. 16. Fig. 15 and Fig. 16 are top views, and the first substrate 701 is omitted in Fig. 15 and Fig. 16 to make the operation of the mechanism easily understandable.

Fig. 15 is a diagram when a voltage is applied. The light controlling means 301 makes a contact with the spacer 805, and is at the first stationary position overlapping with the second aperture 802 formed in the second substrate 801. The third aperture 302 formed in the light controlling mean 301 becomes the optical aperture of the light controlling apparatus in this state. Moreover, the ion-conducting actuator 501 is fixed to the electrode 502, and is coupled with the drive shaft 809 protruded from the groove 904 which is formed in the transmission member 901 via the coupling member 503.

Next, when supplying the electric power to the ion-conducting actuator 501 is stopped, the curvature of the circular arc shape of the ion-conducting actuator 501 changes due to a restoring force of the ion-conducting actuator 501, and as a result, the ion-conducting actuator 501 is displaced. As shown in Fig. 16, the transmission member 901 coupled with the ion-conducting actuator 501 rotates around the rotating shaft 804 formed on the second substrate 801, as a center, and the light controlling means 301 coupled with the drive shaft 902 formed on the transmission member 901 rotates around the rotating shaft 803 formed on the second substrate 801, as a center. Rotational movement of the light controlling means 301 which has rotated stops when the light controlling means 301 makes a contact with the spacer 806, and the light controlling means 301 stops at the second position which is a position retracted completely from the second aperture 802. In this state,' although it is not shown in the diagram, the first aperture 702 formed in the first substrate 701 becomes the optical aperture of the light controlling apparatus.

Even in the second embodiment, the front end of the coupling member 503 is formed to be ring-shaped, and is coupled with the drive shaft 809 protruded from the transmission member 901. It is possible to achieve a similar effect provided that the shape of the front end of the coupling member 503 is a shape which can be hooked to the drive shaft 809, such as a shape in which a notch is formed in a rectangle, and not only the ring shape.

In the second embodiment, similarly as in the first embodiment, since the transmission member 901 which transmits upon widening the amount of displacement to the light controlling means 301 is coupled, it is possible to reduce substantially the amount of displacement of the ion-conducting actuator 501 which is necessary for opening and closing the light controlling means 301. Moreover, in the second embodiment, in addition to that, the drive shaft 809 which is coupled with the ion-conducting actuator 501 is movable not only in a direction (circumferential direction) in which the transmission member 901 is rotated, but also along the groove 904 in a radial direction formed in the transmission member 901.

Normally, the displacement of the ion-conducting actuator 501 having the circular arc shape includes not only a displacement in the circumferential direction but also a displacement in a radial direction. In the second embodiment, the movement in the radial direction of the ion-conducting actuator 501 is not regulated. Furthermore, it is possible to impart a degree of freedom to the disposing the arrangement of the ion-conducting actuator 501.

Even in the second embodiment, the stacked structure is manufactured by the plating process and the etching process.

As it has been described above, the transmission member which transmits upon widening the displacement of the ion-conducting actuator is provided, and this transmission member is coupled with the light controlling means. In other words, it is possible to reduce substantially the displacement of the ion-conducting actuator which rotates the light controlling means. Consequently, it is possible to reduce substantially the voltage to be supplied to the ion-conducting actuator, and to improve reliability of the ion-conducting actuator.

Moreover, by letting the stacked structure to be made of the plating layer, there is shown an effect that it is possible to have a complicated structure as well as to make a size small.

As it has been described above, the light controlling apparatus according to the present invention is useful in an endoscope in which a solid image pickup element is used, and particularly is suitable for a small-size light controlling apparatus to be used in an endoscope of a thin diameter.

According to the present invention, it is possible to provide an ultra-small light controlling apparatus in which an opening and closing operation of a diaphragm blade is possible by a small displacement of an actuator.

## Claims

1. A light controlling apparatus comprising:
a substrate in which, an optical aperture is formed;
a light controlling mechanism which is provided with another optical aperture and a shielding section;
an actuator which generates a power which rotates the light controlling mechanism;
a controlling mechanism which displaces the actuator; and
a transmission member of which, one end is coupled with the light controlling mechanism, and the other end is coupled with the actuator via a coupling member, and which transmits the power of the actuator to the light controlling mechanism,
wherein
the transmission member, at the time of transmitting the power, widens the displacement of the actuator.

2. The light controlling apparatus according to claim 1,
wherein the transmission member is disposed between an outer circumferential portion of the optical aperture formed in the substrate and an outer circumferential portion of the substrate.

3. The light controlling apparatus according to claim 2,
wherein both ends of the transmission member are disposed at positions face-to-face, sandwiching the optical aperture formed in the substrate.

4. The light controlling apparatus according to claim 3,
wherein all components other than the actuator, the controlling mechanism, and the coupling member are a stacked structure made of a plating layer.

5. The light controlling apparatus according to claim 4,
wherein a frame member has an aperture which is larger than the optical aperture formed in the substrate, and the stacked structure is covered by the frame member which is larger than an outer diameter of the substrate.

6. The light controlling apparatus according to claim 5,
wherein
the actuator is an actuator which has a circular arc shape formed by an elastic member, and which changes a chord length thereof by the controlling mechanism, and
the transmission member with which the actuator is coupled is driven by the change in the chord length of the actuator, and the light controlling apparatus which is coupled with the transmission member is rotated.

7. The light controlling apparatus according to claim 6,
wherein
by the rotation of the light controlling mechanism having the other optical aperture, the other optical aperture moves to a first stationary position which overlaps with a position of the optical aperture formed in the substrate, and a second stationary position which is a position retracted from the position of the optical aperture formed in the substrate, and
switches to the optical aperture formed in the substrate and the other optical aperture formed in the light controlling mechanism.

8. The light controlling apparatus according to claim 6,
wherein
the actuator having the circular arc shape is formed of a high-molecular material containing ions, and includes a pair of electrodes, on a surface of a central side of the circular arc, and on a surface facing the surface of the central side of the circular arc, and
a voltage is applied between the pair of electrodes by the controlling mechanism, and the chord length thereof is changed by moving the ions.

9. The light controlling apparatus according to claim 5,
wherein
by the rotation of the light controlling mechanism having the other optical aperture, the other optical aperture moves to a first stationary position which overlaps with a position of the optical aperture formed in the substrate, and a second stationary position which is a position retracted from the position of the optical aperture formed in the substrate, and
switches to the optical aperture formed in the substrate and the optical aperture formed in the light controlling mechanism.
